# EUROPÄISCHE PATENTSCHRIFT

(11) **EP 3 463 553 B1**
(45) Veröffentlichungstag und Bekanntmachung des Hinweises auf die Patenterteilung: **18.03.2020**
(21) Anmeldenummer: 17726918.0
(22) Anmeldetag: 26.05.2017
(51) Int. Cl.: A61N 1/05, A61M 5/158

(54) **UNIPOLAR-KANÜLE**
MONOPOLAR CANNULA
CANULE UNIPOLAIRE

(30) Priorität: 06.06.2016 DE 102016110379
(43) Veröffentlichungstag der Anmeldung: 10.04.2019
(73) Patentinhaber: Pajunk GmbH Medizintechnologie, 78187 Geisingen (DE)
(72) Erfinder: PAJUNK-SCHELLING, Simone, 78187 Geisingen (DE); HAUGER, Martin, 78166 Donaueschingen (DE)
(74) Vertreter: Westphal, Mussgnug & Partner Patentanwälte mbB
(86) Internationale Anmeldenummer: PCT/EP2017/062791
(87) Internationale Veröffentlichungsnummer: WO 2017/211601

(56) Entgegenhaltungen:
- US-A- 3 828 780
- US-A- 4 824 433
- US-A- 5 605 539
- US-A- 5 853 393
- US-A1- 2007 250 021
- US-A1- 2008 058 757
- US-B1- 6 456 874
- US-B2- 7 120 487

## Beschreibung

Die Erfindung betrifft eine Unipolar-Kanüle gemäß Anspruch 1.

Bei den aus dem Stand der Technik bekannten Unipolarkanülen wird die Position der distalen Kanülenspitze, z. B. an einem Nerv durch elektrische Stimulationsreize festgestellt. Die elektrischen Stimulationsimpulse werden über ein Stimulationskabel zugeführt, welches das elektrisch leitende Kanülenrohr im Bereich eines proximalen Ansatzes der Kanüle kontaktiert. Solche Unipolar-Kanülen sind z.B. aus der US 7,022,115 B1 und der
EP 1 002 500 A1 bekannt und werden insbesondere in der Anästhesie für die periphere Nervenblockade verwendet. Relevante Dokumente des Stands der Technik sind auch US-A-2008/058757, US-B-7 120 487, US-B-6 456 874.

Bei diesen bekannten Unipolar-Kanülen ist das Stimulationskabel fest mit der Kanüle verbunden, indem die Isolationshülle des Stimulationskabels mit dem aus Kunststoff bestehenden Ansatz verbunden ist und die elektrisch leitende Ader des Stimulationskabels in den Ansatz eingegossen ist und im Inneren des Ansatzes das metallische Kanülenrohr kontaktiert.

In neuerer Zeit kommt vermehrt Ultraschall zum Einsatz, um die Position der Kanüle bei der Anästhesie zu beobachten und zu prüfen. Dabei wird ein Ultraschallkopf auf die Körperoberfläche aufgesetzt, der Ultraschallsignale aussendet und die von der metallischen Kanüle reflektierten Ultraschallsignale empfängt. Die verbesserte Ultraschall-Sichtbarkeit der Kanüle, wie sie beispielsweise in der WO 2010/012023 A1 beschrieben ist, hat zur Folge, dass die Anästhesisten sich zunehmend auf die Ultraschall-Ortung der Kanüle beschränken und auf die Elektrostimulation verzichten.

Der Erfindung liegt die Aufgabe zu Grunde, diesem Trend zu entsprechen und eine Kanüle zur Verfügung zu stellen, die in zweckmäßiger Weise die Möglichkeiten der Ortung der Kanülenposition mittels Ultraschall und mittels Elektrostimulation vereinigt.

Diese Aufgabe wird erfindungsgemäß gelöst durch eine Unipolar-Kanüle mit den Merkmalen des Anspruchs 1.

Vorteilhafte Ausführungsformen der Erfindung sind in den rückbezogenen Unteransprüchen angegeben.

Der wesentliche Gedanke der Erfindung besteht darin, dass das Stimulationskabel nicht fest mit der Kanüle verbunden ist, sondern optional an den Ansatz der Kanüle angebracht werden kann. Wird die Unipolar-Kanüle lediglich mit einer Ultraschall-Positionierung verwendet, so bleibt das Stimulationskabel unbenutzt und von der Kanüle getrennt bzw. ein an der Kanüle angebrachtes Stimulationskabel wird von der Kanüle abgenommen. Das Stimulationskabel ist dadurch nicht störend und die Kanüle kann wie eine herkömmliche Kanüle ohne Elektrostimulation verwendet werden. Soll eine Positionierung mittels Elektrostimulation erfolgen, so kann dieselbe Kanüle verwendet werden, wobei lediglich das Stimulationskabel seitlich an dem Ansatz angebracht wird bzw. ein bereits an der Kanüle angebrachtes Stimulationskabel an der Kanüle verbleibt und dabei die leitende Ader des Stimulationskabels zwangsweise das metallische Kanülenrohr kontaktiert. Dieselbe Kanüle kann auf diese Weise alternativ mit
oder ohne Elektrostimulation verwendet werden.

Zur Befestigung des Stimulationskabels an dem Ansatz der Kanüle dient ein Befestigungsteil aus einem elektrisch isolierenden Kunststoff, welches seitlich an dem Ansatz so befestigbar ist, dass es sich an dessen Außenumfang anlegt. Das Befestigungsteil nimmt ein elektrisch leitendes Kontaktteil auf, welches mit der Ader des Stimulationskabels elektrisch leitend verbunden ist. Das Kontaktteil und die Ader können fest verbunden sein, z. B. durch eine Lötverbindung, oder steckbar miteinander verbindbar sein. Wird das Befestigungsteil an dem Ansatz angebracht, so greift das Kontaktteil in eine Aussparung des Ansatzes, die das metallische Kanülenrohr frei lässt, sodass das Kontaktteil das Kanülenrohr leitend kontaktieren kann. Das Befestigungsteil überdeckt dabei das Kontaktteil an der Außenseite vollständig, wodurch das Kontaktteil und seine Verbindung mit der Ader des Stimulationskabels am Außenumfang des Ansatzes gegen eine Berührung durch den Benutzer isoliert sind. Die konstruktiven Maßnahmen zur Befestigung des Stimulationskabels und zum Kontaktieren des Kanülenrohres beeinflussen auf diese Weise die äußere Gestaltung des Ansatzes nicht, wodurch die Handhabung der Kanüle ohne Stimulationskabel unbehindert ist und vollständig einer herkömmlichen Kanüle ohne Elektrostimulation entspricht.

In einer bevorzugten Ausführung kann die Befestigung des Stimulationskabels mit einem Befestigungsteil erfolgen, welches mit einer Schnappverbindung an dem Ansatz angebracht wird. Die Schnappverbindung kann dabei beispielsweise durch federnde Rastarme des Befestigungsteils bewirkt werden, die an dem Ansatz einrasten. In einer Ausführung ist das Kontaktteil vorzugsweise zwischen einem oder mehreren Paaren von Rastarmen angeordnet. Die Rastarme legen sich dabei diametral zueinander beiderseits an den Ansatz an, wobei die Aussparung für das Eingreifen des Kontaktteils sich in dem Bereich des Ansatzes zwischen den Rastarmen befindet. In einer anderen Ausführung wird das Befestigungsteil schwenkbar an dem Ansatz eingehängt und rastet an dem Ansatz ein, wenn es gegen diesen geschwenkt wird.

Das Kontaktteil zum elektrischen Kontaktieren des Kanülenrohres kann in unterschiedlicher Weise ausgebildet sein, wobei lediglich gewährleistet sein muss, dass das Kontaktteil einen guten zuverlässigen elektrischen Kontakt mit dem Kanülenrohr bewirkt, wenn das Befestigungsteil an dem Ansatz angebracht ist. In einer Ausführung kann das Kontaktteil als Schneid-Klemm-Kontakt ausgebildet sein, der gabelförmig radial auf das Kanülenrohr gedrückt wird. Die beiden Schenkel des Schneid-Klemm-Kontaktes drücken sich dabei in die Umfangsmantelfläche des Kanülenrohres ein, um den erforderlichen Kontaktdruck zu bewirken.

In einer anderen Ausführung kann das Kontaktteil als Federzunge ausgebildet sein, die sich bei aufgesetztem Befestigungsteil unter elastischem Federdruck außen an das Kanülenrohr anlegt.

Der proximale Ansatz der Unipolar-Kanüle weist einen Anschluss auf, durch welchen eine Flüssigkeit, insbesondere ein Anästhetikum in das Kanülenrohr eingeleitet werden kann, um diese Flüssigkeit über die distale Austrittsöffnung der Kanüle zu applizieren. Der Anschluss ist vorzugweise koaxial zu dem Kanülenrohr ausgebildet, was durch das seitliche Anbringen des Stimulationskabels ermöglicht wird. Der Anschluss kann beispielsweise als Konnektor, insbesondere Luer-Lock-Konnektor, ausgebildet sein. Dieser Konnektor kann unmittelbar an dem Ansatz ausgebildet sein, wie dies z. B. in der US 7,022,115 B1 gezeigt ist. Alternativ kann der Konnektor an einem Schlauch angebracht sein, der koaxial an dem Ansatz angesetzt ist, wie dies z. B. in der EP 1 002 500 A1 gezeigt ist.

Im Folgenden wird die Erfindung anhand von in der Zeichnung dargestellten Ausführungsbeispielen näher erläutert. Es zeigen:
- Fig.1: eine perspektivische Ansicht der Unipolar-Kanüle mit befestigtem Stimulationskabel in einer ersten Ausführung,
- Fig. 2: in entsprechender perspektivischer Darstellung die Unipolar-Kanüle mit abgenommenem Stimulationskabel,
- Fig. 3: eine teilweise axial geschnittene Seitenansicht der Unipolar-Kanüle mit befestigtem Stimulationskabel,
- Fig. 4: eine Draufsicht auf die Unipolar-Kanüle,
- Fig. 5: einen Querschnitt durch die Unipolar-Kanüle mit befestigtem Stimulationskabel gemäß der Schnittlinie F-F in Figur 3,
- Fig. 6: eine perspektivische Ansicht der Unipolar-Kanüle mit befestigtem Stimulationskabel in einer zweiten Ausführung,
- Fig. 7: eine entsprechende perspektivische Ansicht der Unipolar-Kanüle in der zweiten Ausführung mit abgenommenem Stimulationskabel,
- Fig. 8: in einer teilweise axial geschnittenen Seitenansicht die Unipolar-Kanüle in der zweiten Ausführung mit abgenommenem Stimulationskabel,
- Fig. 9: in einer axial teilgeschnitten Seitenansicht die Unipolar-Kanüle mit befestigtem Stimulationskabel,
- Fig. 10: in einer Draufsicht die Unipolarkanüle in der zweiten Ausführung mit befestigtem Stimulationskabel,
- Fig. 11: einen Querschnitt durch die Unipolar-Kanüle mit befestigtem Stimulationskabel gemäß der Schnittlinie F-F in Figur 9,
- Fig. 12: eine perspektivische Ansicht der Unipolar-Kanüle mit befestigtem Stimulationskabel in einer dritten Ausführung,
- Figur 13: eine perspektivische Ansicht dieser dritten Ausführung mit abgenommenen Stimulationskabel,
- Figur 14: eine Seitenansicht der Unipolarkanüle in der dritten Ausführung mit abgenommenen Stimulationskabel,
- Figur 15: einen Schnitt gemäß der Schnittlinie A-A in Figur 14,
- Figur 16: eine teilweise axial geschnittene Seitenansicht der Unipolar-Kanüle in der dritten Ausführung mit befestigtem Stimulationskabel,
- Figur 17: einen Querschnitt gemäß der Schnittlinie B-B in Figur 16,
- Figur 18: eine teilweise axial geschnittene Seitenansicht der Unipolar-Kanüle in einer vierten Ausführung,
- Figur 19: eine Axialansicht der Unipolar-Kanüle der Figur 18 von der proximalen Seite,
- Figur 20: eine entsprechend Figur 18 teilweise axial geschnittene Seitenansicht der Unipolar-Kanüle in der vierte Ausführung mit befestigtem Stimulationskabel,
- Figur 21a, b und c: die Befestigung des Stimulationskabels an der Unipolarkanüle in der vierten Ausführung in drei Schritten,
- Figur 22: eine teilweise axial geschnittene Seitenansicht der Unipolarkanüle in einer fünften Ausführung mit abgenommenen Stimulationskabel,
- Figur 23: eine axiale Ansicht der Unipolarkanüle in der Stellung der Figur 22 von der proximalen Seite,
- Figur 24: eine entsprechende axiale Ansicht von der distalen Seite,
- Figur 25: eine teilweise axial geschnittene Seitenansicht der Unipolarkanüle in der fünften Ausführung mit befestigtem Stimulationskabel,
- Figur 26a, b und c: das Befestigen des Stimulationskabels an der Unipolar-Kanüle der fünften Ausführung in drei Schritten,
- Fig. 27: eine Seitenansicht der Unipolarkanüle mit befestigtem Stimulationskabel in einer sechsten Ausführung,
- Fig. 28: eine Draufsicht auf die sechste Ausführung,
- Fig. 29: einen Schnitt gemäß der Linie A-A in Figur 27,
- Fig. 30: eine Explosionsdarstellung der Unipolar-Kanüle mit Stimulationskabel gemäß der sechsten Ausführung,
- Fig. 31: eine perspektivische Ansicht der Unipolar-Kanüle mit angesetztem Stimulationskabel in der sechsten Ausführung und
- Fig. 32: einen Axialschnitt der Unipolar-Kanüle mit angesetztem Stimulationskabel gemäß der sechsten Ausführung.

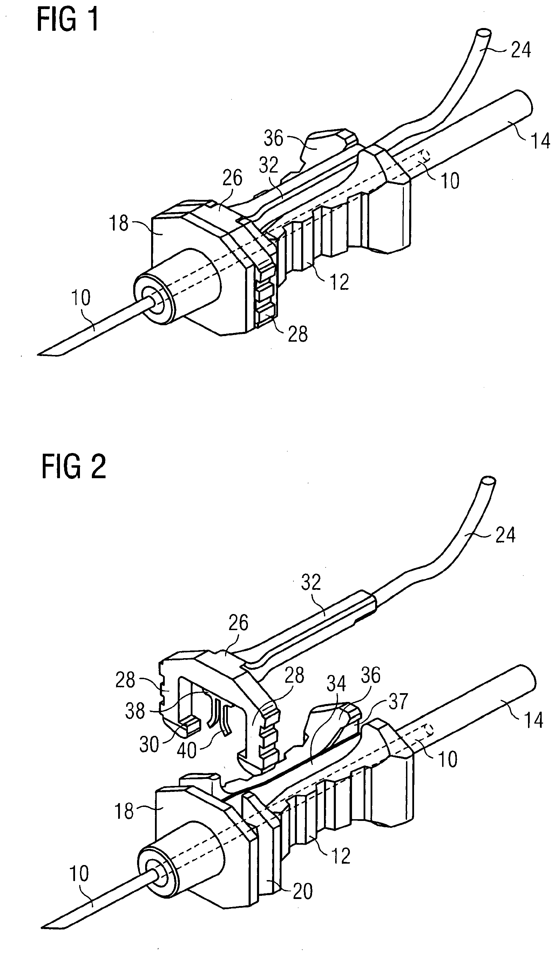

In den Figuren 1 bis 5 ist ein erstes Ausführungsbeispiel der Unipolar-Kanüle gemäß der Erfindung dargestellt.

Die Unipolar-Kanüle weist ein metallisches Kanülenrohr 10 auf, welches an seinem Außenumfang mit einem elektrisch isolierenden Kunststoff beschichtet ist, der nur die distale Spitze des metallischen Kanülenrohrs 10 frei lässt. Am proximalen Ende ist das Kanülenrohr 10 in einen Ansatz 12 aus Kunststoff angespritzt. Der Ansatz 12 hat im Wesentlichen die übliche Form eines rechteckigen Quaders, dessen vorderes und hinteres Ende flanschförmig verbreitert ist, um ein sicheres Greifen und Führen der Kanüle mit zwei Fingern zu ermöglichen. Das Kanülenrohr 10 führt axial durch den Ansatz 12 hindurch. Das proximal aus dem Ansatz 12 herausragenden Ende des Kanülenrohres 10 ist von einem koaxialen Schlauch 14 umschlossen, der abgedichtet an dem Ansatz 12 angebracht ist. Der Schlauch 14 dient als Zuspritzschlauch, durch welchen eine Flüssigkeit, z. B. ein Anästhetikum in das Kanülenrohr 10 eingeleitet werden kann, welches dann distal aus dem Kanülenrohr 10 austritt. Der Schlauch 14 weist an seinem in der Zeichnung nicht dargestellten freien Ende einen Anschluss auf, z. B. einen Luer-Lock-Anschluss, an welchen eine Spritze oder dergleichen zum Zuführen der Flüssigkeit angeschlossen werden kann. Alternativ kann der Anschluss auch unmittelbar an dem Ansatz 12 angeordnet sein, wenn kein Zuspritzschlauch erwünscht ist.

Der Ansatz 12 weist in seinem distalwärtigen Abschnitt eine Aussparung 16 auf, die in der in der Zeichnung oberen Seitenfläche des Ansatzes 12 offen ist und in der Tiefe zumindest bis zu dem Kanülenrohr 10 reicht. Im dargestellten Ausführungsbeispiel ist die Aussparung 16 diametral durchgehend durch den Ansatz 12 offen. Das Kanülenrohr 10 ist in seinem innerhalb der Aussparung 16 verlaufenden axialen Abschnitt blank, d. h. das metallische Kanülenrohr 10 weist in diesem Abschnitt keine isolierende Umfangsbeschichtung auf. In dem vorderen distalen verbreiterten Flansch 18 des Ansatzes 12 ist eine in Umfangsrichtung verlaufende Nut 20 ausgebildet. Die Nut 20 ist diametral beiderseits der Aussparung 16 ausgebildet, wobei die Aussparung 16 distalwärts bis in die Nut 20 hineinreicht. An dem in der Zeichnung unteren Ende weist die Nut 20 beiderseits jeweils eine Innenstufe 22 auf.

Soll die Unipolar-Kanüle für die Elektrostimulation verwendet werden, so wird an dem Ansatz 12 ein Stimulationskabel 24 befestigt, welches an ein elektrisches Stimulationsgerät anschließbar ist. Das Stimulationskabel 24 weist ein Befestigungsteil 26 auf, welches aus einem elektrisch isolierenden Kunststoff besteht und an die Isolierung des Stimulationskabels anschließt. Das Befestigungsteil 26 ist mittels einer Schnappverbindung an dem Ansatz 12 befestigbar. Die Schnappverbindung wird in dem dargestellten Ausführungsbeispiel durch ein Paar von federnden Rastarmen 28 des Befestigungsteils 26 gebildet. Die Rastarme 28 sind als U-förmige Schenkel ausgebildet, die so geformt sind, dass sie in die Nut 20 eingefügt werden können und dabei diametral den Ansatz 12 im Bereich der Nut 20 beiderseits der Aussparung 16 umgreifen. An den freien Enden der Rastarme 28 ist jeweils eine nach innen gerichtete Nase 30 ausgebildet. Wird das Befestigungsteil 26 mit den Rastarmen 28 seitlich in radialer Richtung auf den Ansatz 12 aufgesetzt, so werden die Rastarme 28 in die Nut 20 eingeschoben und schnappen mit ihrer jeweiligen Nase 30 hinter der jeweiligen Innenstufe 22 ein. Dadurch wird das Befestigungsteil 26 auf dem Ansatz 12 aufgeklipst und wird an dem Ansatz 12 gehalten. Das Befestigungsteil 26 mit den Rastarmen 28 fügt sich dabei in die Außenkontur des vorderen Flansches 18 ein. Ein proximalwärts gerichteter Steg 32 des Befestigungsteils 26 fügt sich bei aufgeklipstem Befestigungsteil 26 in eine Rille 34 ein, die axial in der in der Zeichnung oberen Seitenfläche des Ansatzes 12 verläuft. Der proximale hintere Flansch 36 des Ansatzes 12 weist in Fortsetzung der Rille 34 eine Kerbe 37 auf, in welche der Steg 32 einrastet. Dadurch ist das Befestigungsteil 26 im eingerasteten Zustand über die gesamte axiale Länge des Ansatzes 12 an diesem Ansatz 12 fixiert.

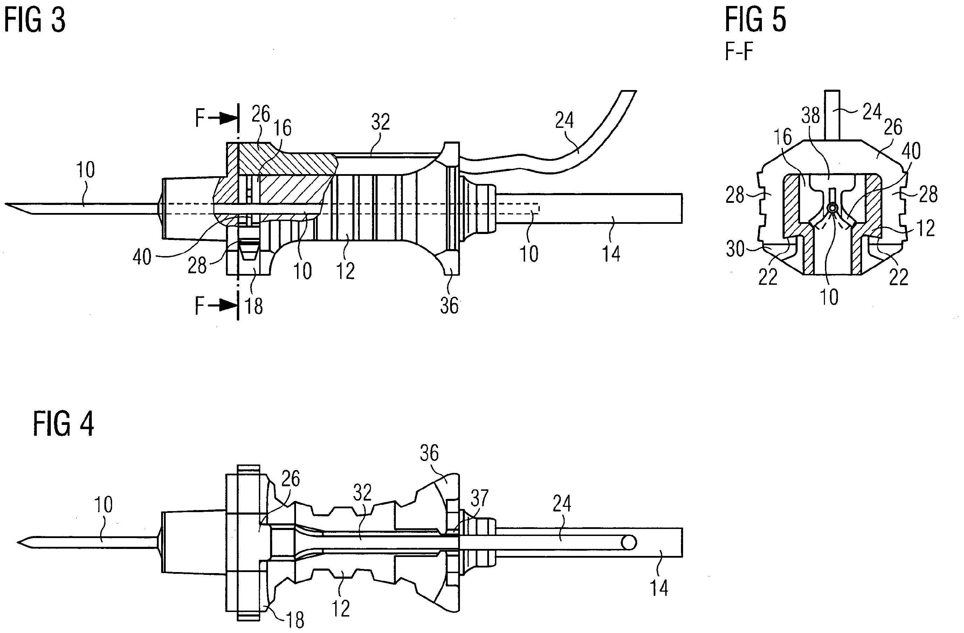

Die elektrisch leitende Ader des Stimulationskabels 24 ist mit einem in dem Befestigungsteil 26 angeordneten metallischen Kontaktteil 38 elektrisch leitend verbunden. Das Kontaktteil 38 ist in dem ersten Ausführungsbeispiel als ein Schneid-Klemm-Kontakt 40 ausgebildet. Der Schneid-Klemm-Kontakt 40 liegt mittig zwischen den Rastarmen 28 in der durch die Rastarme 28 aufgespannten Ebene und verläuft im Wesentlichen parallel zu den Rastarmen 28. Wird das Befestigungsteil 26 mit den Rastarmen 28 auf den Ansatz 12 aufgeschnappt, so dringt der Schneid-Klemm-Kontakt 40 radial in die Aussparung 16 ein und wird mit seinen beiden gabelförmigen Schenkel auf das blanke Kanülenrohr 10 gedrückt. Die Schenkel des Schneid-Klemm-Kontaktes 40 drücken sich dabei in die Oberfläche des metallischen Kanülenrohres 10 ein, wie dies insbesondere in Figur 5 zu erkennen ist. Dadurch wird bei aufgeschnapptem Befestigungsteil 26 ein zuverlässiger elektrischer Kontakt von der Ader des Stimulationskabels 24 über den Schneid-Klemm-Kontakt 40 zu dem Kanülenrohr 10 hergestellt. Dabei deckt das Befestigungsteil 26 das Kontaktteil 38 am Außenumfang des Ansatzes 12 vollständig isolierend ab.

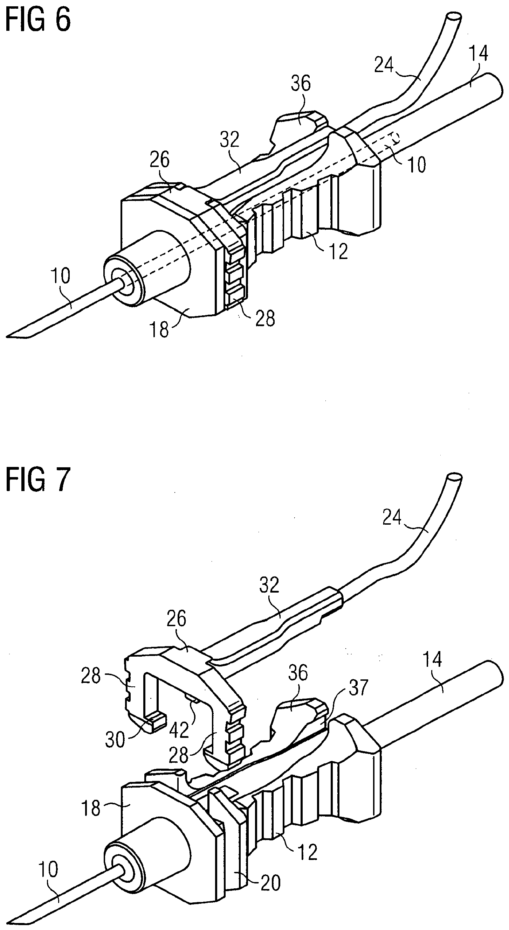

Ein zweites Ausführungsbeispiel der Erfindung ist in den Figuren 6 bis 11 gezeigt. Soweit dieses Ausführungsbeispiel mit dem ersten Ausführungsbeispiel übereinstimmt, sind dieselben Bezugszahlen verwendet und die vorangehende Beschreibung trifft auch für dieses zweite Ausführungsbeispiel zu.

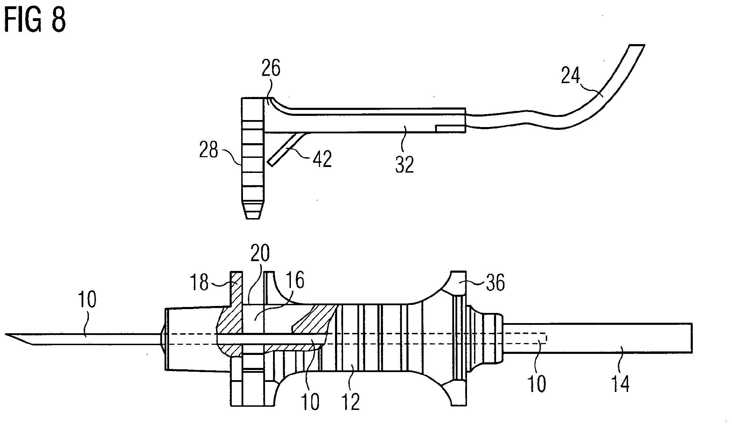

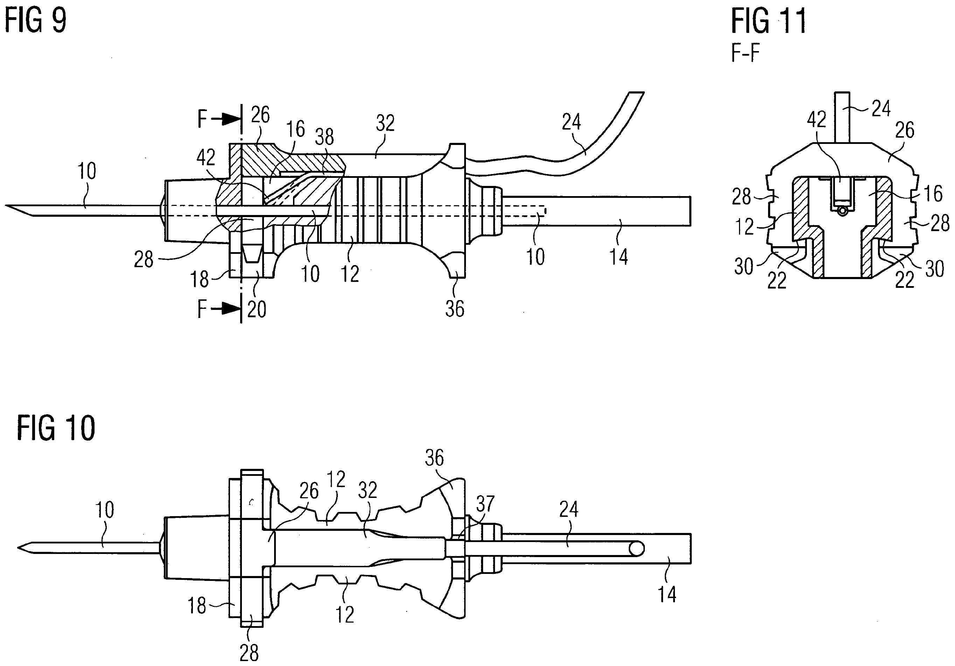

Das zweite Ausführungsbeispiel unterscheidet sich von dem ersten Ausführungsbeispiel in der Ausbildung des Kontaktteils 38. Das Kontaktteil 38 weist in dieser zweiten Ausführung eine Federzunge 42 auf, die aus einem leitenden federnden Metall besteht und mit der Ader des Stimulationskabels 24 leitend verbunden ist. Die Federzunge 42 ist in dem Kunststoff des Befestigungsteils 26 so festgelegt, dass sie distalwärts nach innen von dem Steg 32 des Befestigungsteils 26 abgespreizt ist, wie dies insbesondere in Figur 8 zu sehen ist. Wird das Befestigungsteil 26 mittels der Rastarme 28 auf den Ansatz 12 aufgeschnappt, so greift die Federzunge 42 in die Aussparung 16 ein und legt sich unter elastischer Federspannung am Außenumfang des blanken Kanülenrohrs 10 an, wie dies in den Figuren 9 und 11 zu sehen ist. Die Federzunge 42 stellt auf diese Weise einen zuverlässigen elektrischen Kontakt zwischen dem Kontaktteil 38 sowie mit der mit dem Kontaktteil 38 verbundenen Ader des Stimulationskabels 24 und dem Kanülenrohr 10 her.

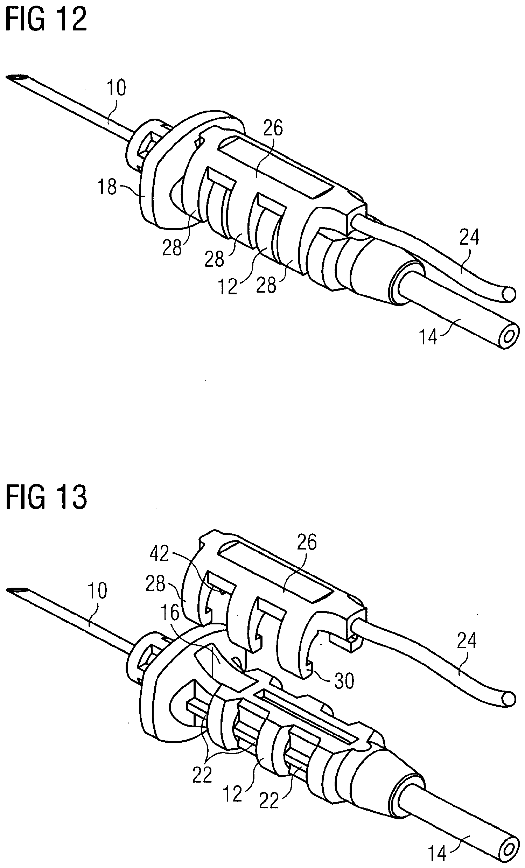

In den Figuren 12 bis 17 ist eine dritte Ausführung der Unipolar-Kanüle gemäß der Erfindung dargestellt. Soweit diese Ausführung mit den vorangehend beschriebenen ersten und zweiten Ausführungen übereinstimmt sind dieselben Bezugszahlen verwendet und die vorangehende Beschreibung trifft auch für diese dritte Ausführung zu.

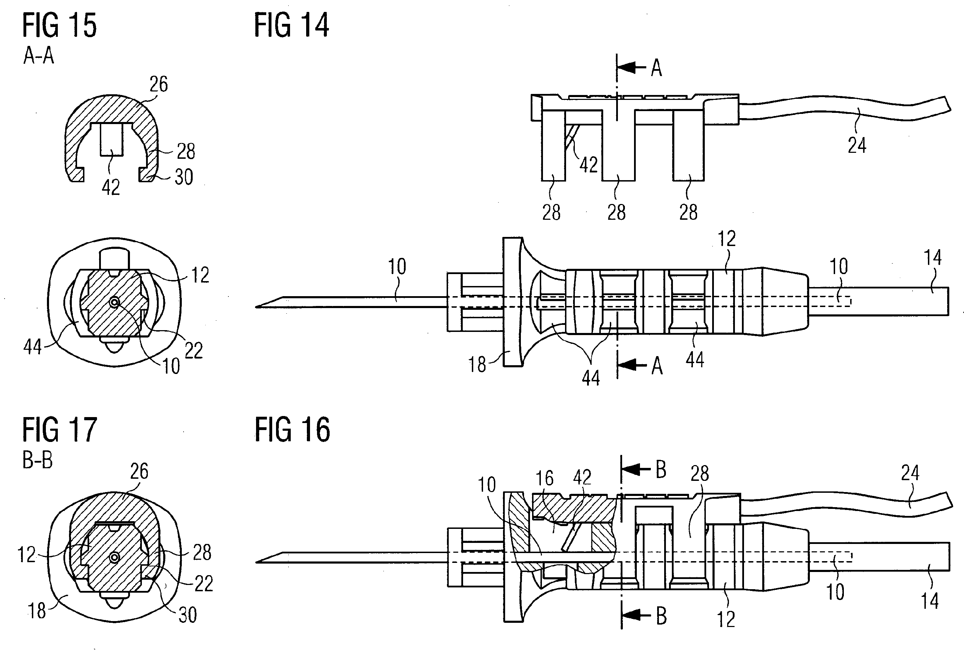

Die dritte Ausführung unterscheidet sich von der ersten und der zweiten Ausführung im Wesentlichen in der Ausbildung der Schnappverbindung zwischen dem Ansatz 12 und dem Befestigungsteil 26. In dieser dritten Ausführung weist das Befestigungsteil 26 drei Paare von Rastarmen 28 auf, die in axialer Richtung beabstandet sind. Die jeweiligen Paare von Rastarmen 28 umgreifen den Ansatz 12 und fügen sich bei Befestigung des Stimulationskabels in entsprechend axial beabstandete Nuten 44 im Umfang des Ansatzes 12 ein. Dabei hintergreifen nach innen gerichtete Nasen 30 der jeweiligen Rastarme 28 in diesen Nuten 44 angeordnete Innenstufen 22. Wird das Befestigungsteil 26 auf den Ansatz 12 aufgesetzt, so rastet das Befestigungsteil 26 auf diese Weise mit den drei Paaren von Rastarmen 28 hinter die Innenstufen 22, wodurch das Befestigungsteil 26 mit dem Stimulationskabel 24 über die ganze axiale Länge des Ansatzes 12 an diesem Ansatz 12 fixiert ist. Das distal vorderste Paar von Rastarmen 28 umgreift dabei den Ansatz 12 im Bereich der Aussparung 16. Dadurch kommt das zwischen den distal vordersten Rastarmen 28 angeordnete Kontaktteil 38 in der Aussparung 16 mit dem Kanülenrohr 10 in elektrisch leitenden Kontakt, wie dies in Figur 16 zu sehen ist. Das Kontaktteil 38 kann als Federzunge 42 ausgebildet sein, wie dies insbesondere in den Figuren 15 und 16 zu sehen ist. Selbstverständlich kann auch in dieser dritten Ausführungsform das Kontaktteil 38 als Schneid-Klemm-Kontakt 40 ausgebildet sein, wie dies in der ersten Ausführung beschrieben ist.

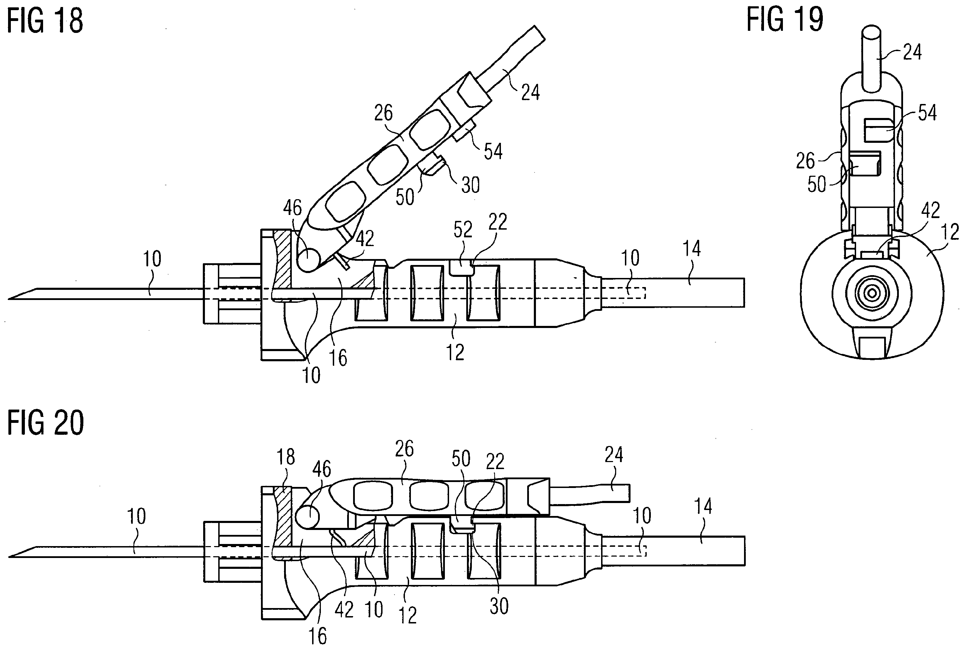

In den Figuren 18 bis 21 ist eine vierte Ausführung der erfindungsgemäßen Unipolar-Kanüle dargestellt. Soweit diese vierte Ausführung mit den vorangehend beschriebenen Ausführungen übereinstimmt, sind dieselben Bezugszahlen verwendet und die vorangehende Beschreibung trifft auch für diese dritte Ausführung zu.

Die vierte Ausführung unterscheidet sich von den vorangehenden Ausführungen im Wesentlichen in der Art der Befestigung des Befestigungsteils 26 an dem Ansatz 12.

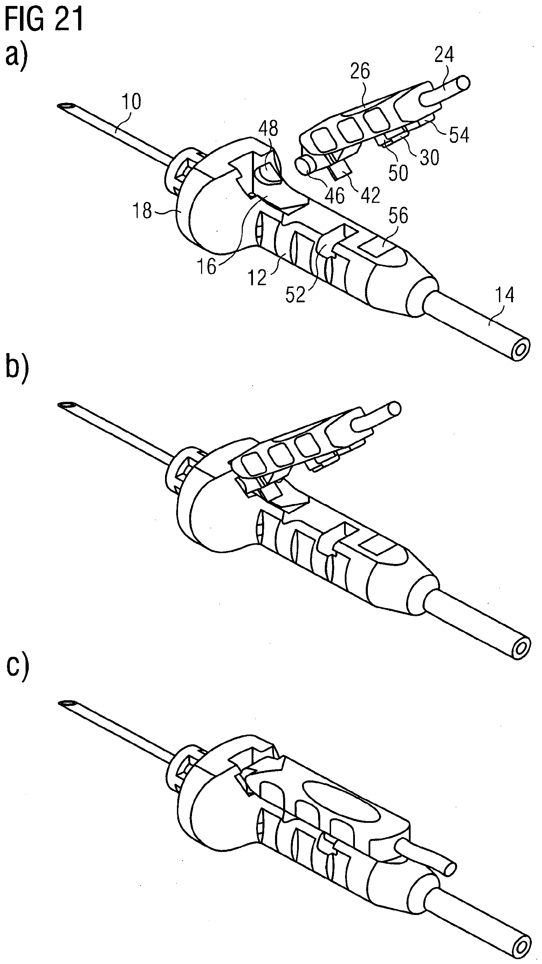

Das Befestigungsteil 26 greift in dieser vierten Ausführung mit seinem distalen Ende in die Aussparung 16 ein. Am distalen Ende des Befestigungsteils 26 sind seitlich, d. h. senkrecht zur Axialrichtung der Kanüle abstehende Lagerzapfen 46 angeformt. Beim Einsetzen des Befestigungsteils 26 in die Aussparung 16, wie dies in den Figuren 21a und 21b gezeigt ist, werden diese kreiszylindrischen Lagerzapfen 46 von einer Lagerpfanne 48 aufgenommen, die in dem vorderen Flansch 18 in dessen proximalwärts gerichteter Wandung ausgebildet ist. Wenn das Befestigungsteil 26 mit seinen Lagerzapfen 46 in der Lagerpfanne 48 sitzt, ist das Befestigungsteil 26 um die quer zur Kanülenachse verlaufende Achse der Lagerzapfen 46 schwenkbar an dem Ansatz 12 angeordnet, wie in den Figuren 18 und 21b dargestellt ist.

Das Befestigungsteil 26 kann nun gegen den Ansatz 12 aus der in Figur 18 gezeigten Stellung in die in Figur 20 gezeigte Stellung verschwenkt werden, wie dies auch in den Figuren 21b und 21c gezeigt ist. Sobald das Befestigungsteil 26 an dem Ansatz 12 anliegt (Figur 20) greift ein Rastarm 50, der an der dem Ansatz 12 zugewandten Fläche des Befestigungsteils 26 angeformt ist in eine Vertiefung 52 in der Außenoberfläche des Ansatzes 12. Ein an dem freien Ende dieses Rastarmes 50 angeordnete Nase 30 rastet dabei hinter einer Innenstufe 22 der Vertiefung 52 ein, wie dies insbeondere in Figur 20 zu sehen ist. Dadurch ist das Befestigungsteil 26 über seine gesamte axiale Länge an dem Ansatz 12 fixiert.

An der dem Ansatz 12 zugewandten Fläche des Befestigungsteils 26 ist außerdem ein Vorsprung 54 angeformt. Wird das Befestigungsteil 26 gegen den Ansatz 12 in die Befestigungsstellung der Figur 20 geschwenkt, so taucht dieser Vorsprung 54 in eine korrespondierende Einsenkung 56 in der Außenoberfläche des Ansatzes 12 ein, wodurch das Befestigungsteil 26 zusätzlich formschlüssig gegen eine Querverschiebung gegenüber dem Ansatz 12 gesichert ist.

Das Kontaktteil 38 ist in der Darstellung der Figuren 18 bis 21 als Federzunge 42 ausgebildet. Selbstverständlich ist auch eine Ausbildung des Kontaktteils 38 als Schneid-Klemm-Kontakt 40 möglich.

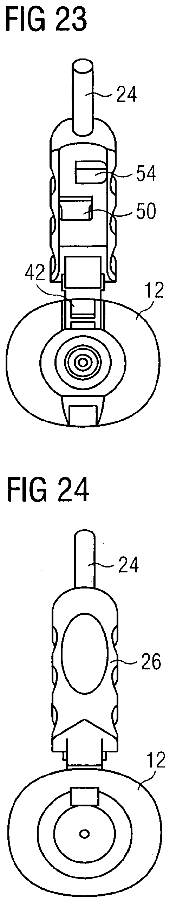

In den Figuren 23 bis 26 ist eine fünfte Ausführung der erfindungsgemäßen Unipolar-Kanüle dargestellt. Soweit diese Ausführung mit den vorangehend beschriebenen Ausführungen übereinstimmt, sind dieselben Bezugszahlen verwendet und die vorangehende Beschreibung gilt auch für diese Ausführung.

Die fünfte Ausführung stimmt im Wesentlichen mit der vierten Ausführung der Figuren 18 bis 21 überein und unterscheidet sich von dieser vierten Ausführung lediglich in der Ausbildung der schwenkbaren Lagerung des Befestigungsteils 26 an dem Ansatz 12.

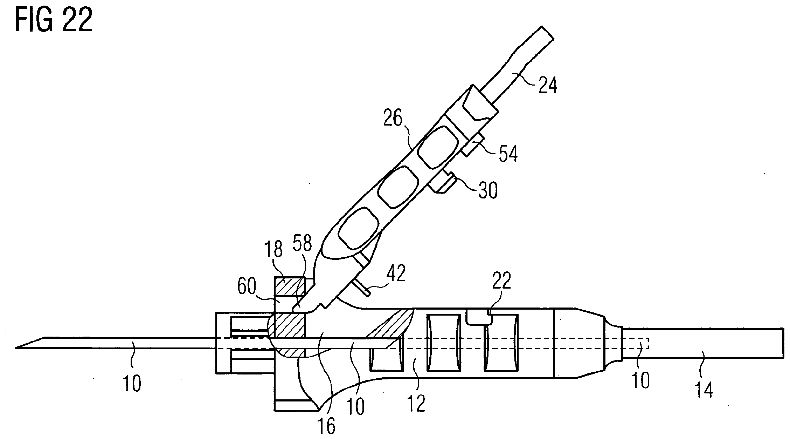

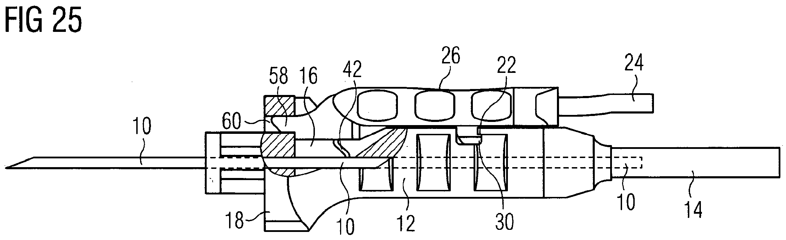

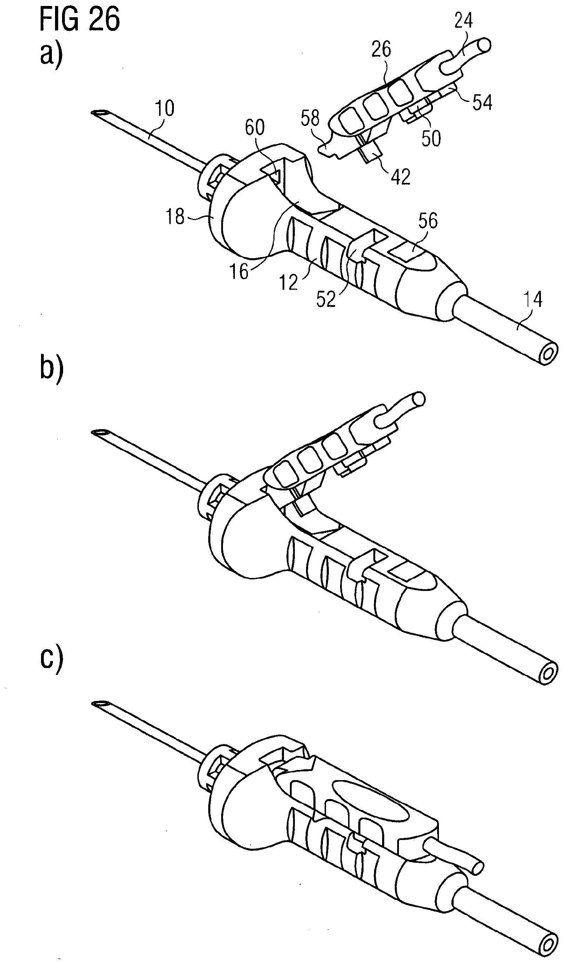

In dieser fünften Ausführung ist anstelle der Lagerzapfen und der Lagerpfanne am distalen Ende des Befestigungsteils 26, welches in die Aussparung 16 eingreift, eine Lagernase 58 angeformt. Wird das Befestigungsteil 26 mit seinem distalen Ende in die Aussparung 16 eingesetzt, so greift die Lagernase 58 in einen Durchbruch 60 ein, der in der proximalwärts gerichteten Stirnfläche des vorderen Flansches 18 ausgebildet ist. Dadurch ist das Befestigungsteil 26 mit seinem distalen vorderen Ende schwenkbar in dem Flansch 18 des Ansatzes 12 gelagert und kann von der Stellung der Figur 22 bzw. Figur 26b in die an dem Ansatz 12 anliegende Stellung der Figur 25 bzw. Figur 26c geschwenkt werden. Wenn das Befestigungsteil 26 bei dieser Schwenkbewegung an dem Ansatz 12 zur Anlage kommt, rastet der Rastarm 50 mit seiner Nase 30 in die Vertiefung 52 mit der Innenstufe 22 ein, wie dies in Verbindung mit der vierten Ausführung beschrieben ist. Dadurch ist die Schnappverbindung hergestellt und das Befestigungsteil 26 mit dem Stimulationskabel 24 an dem Ansatz 12 und damit an der Kanüle fixiert.

Auch in dieser Ausführung kann das Kontaktteil 38 als Federzunge 42 oder als Schneid-Klemm-Kontakt 40 ausgebildet sein.

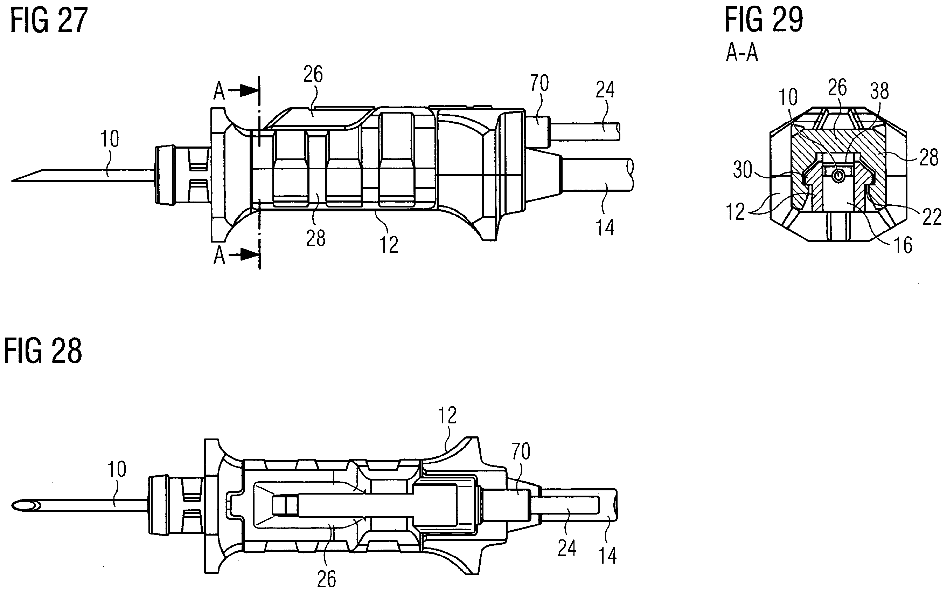

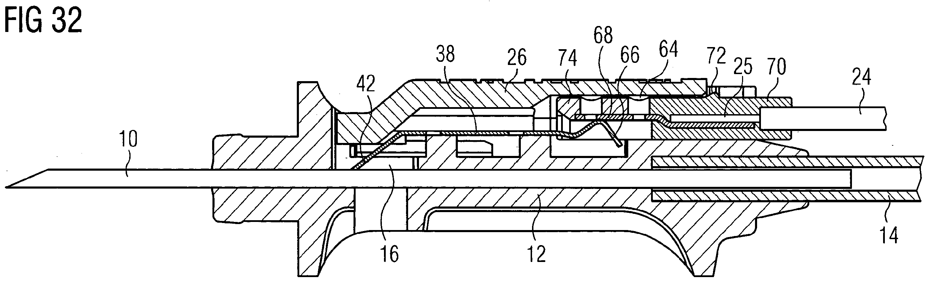

In den Figuren 27-32 ist eine sechste Ausführung der erfindungsgemäßen Unipolar-Kanüle dargestellt. Soweit diese sechste Ausführung mit den vorangehend beschriebenen Ausführungen übereinstimmt, sind dieselben Bezugszahlen verwendet und die vorangehende Beschreibung trifft auch für diese sechste Ausführung zu. Während bei den vorangehenden Ausführungen die Ader 25 des Stimulationskabels 24 elektrisch leitend fest mit dem Kontaktteil 38 verbunden ist, z. B. durch Verlöten, Verschweißen oder dergleichen, ist in der sechsten Ausführung das Stimulationskabel 24 mit seiner leitenden Ader 25 steckbar mit dem Kontaktteil 38 elektrisch leitend verbunden. Im Übrigen entspricht die sechste Ausführung in wesentlichen Teilen der in den Figuren 12-17 dargestellten dritten Ausführung.

Das Befestigungsteil 26 aus Kunststoff ist seitlich auf den Ansatz 12 des Kanülenrohres 10 aufgeschnappt und fügt sich außen in die Umfangskontur des Ansatzes 12 ein. Das Befestigungsteil 26 weist vier Paare von Rastarmen 28 auf, die axial beabstandet an dem Befestigungsteil 26 ausgebildet sind. Die Rastarme 28 umgreifen den Ansatz 12 und schnappen federnd mit nach innen gerichteten Nasen 30 hinter beidseitig an dem Ansatz 12 ausgebildeten Innenstufen 22 ein, um das Befestigungsteil 26 an dem Ansatz 12 zu befestigen.

Das Befestigungsteil 26 nimmt das Kontaktteil 38 auf, welches als Blattfeder ausgebildet ist und mit einem als Federzunge 42 ausgebildeten vorderen Ende in die Aussparung 16 eingreift, um das Kanülenrohr 10 elektrisch leitend zu kontaktieren. Selbstverständlich kann auch hier anstelle einer Federzunge 42 ein Schneid-Klemm-Kontakt an dem Kontaktteil 38 ausgebildet sein. Bei der Montage wird das Kontaktteil 38 in seitliche Führungsnuten in der Innenseite des Befestigungsteils 26 eingeschoben und in diesen Führungsnuten durch abgespreizte Federlappen 62 geklemmt und fixiert.

In der sechsten Ausführung wird das Stimulationskabel 24 steckbar und damit trennbar mit dem Kontaktteil 38 leitend verbunden, um über dieses mittels des Befestigungsteils 26 an dem Ansatz 12 angebrachte Kontaktteil 38 das Kanülenrohr 10 zu kontaktieren. Hierzu weist das Befestigungsteil 26 in seinem hinteren Bereich einen achsparallelen proximalwärts offenen Tunnel 64 auf, der durch eine gegen den Außenumfang geschlossene U-förmige Auswölbung des Befestigungsteils 26 einerseits und durch die Wandung des Ansatzes 12 andererseits gebildet wird. Das Kontaktteil 38 ragt mit einem an seinem proximalen Ende angeformten Federarm 66 in diesen Tunnel 64 hinein. Der Federarm 66 ist nach oben ausgewölbt, sodass seine Auswölbung in den freien Querschnitt des Tunnels 64 hineinragt.

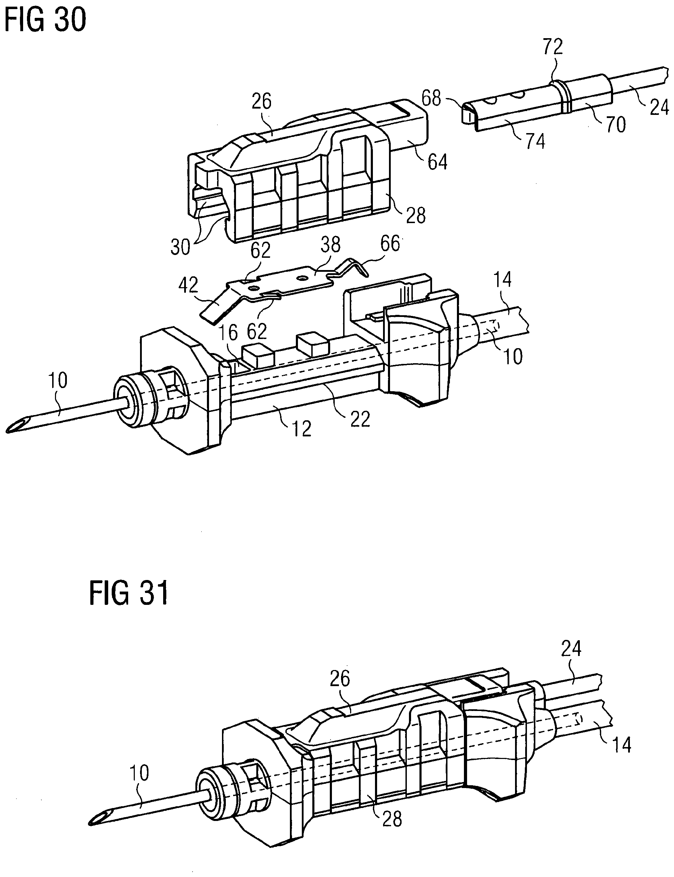

An dem freien Ende der Ader 25 des Stimulationskabels 24 ist elektrisch leitend ein metallischer Kontaktstreifen 68 angebracht, z. B. durch Verlöten, Verschweißen oder dergleichen. Das freie Ende der Ader 25 und der Kontaktstreifen 68 sind mit einer Kunststoffhülse 70 umspritzt. Die Kunststoffhülse 70 entspricht mit ihrem Querschnitt dem Innenquerschnitt des Tunnels 64, sodass die Kunststoffhülse 70 im Querschnitt passend in den Tunnel 64 eingeschoben werden kann, wobei sie mit einem Außenwulst 72 in einer Innennut des U-Profils des Tunnels 64 einrastet. Die Kunststoffhülse 70 umschließt in ihrem rückwärtigen Teil das freie Ende der Ader 25 und den Kontaktstreifen 68 und umgreift auch die Isolation des Stimulationskabels 24. Im proximalen vorderen Ende bildet die Kunststoffhülse 70 ein gegen den Ansatz 12 offenes U-Profil 74, wobei der Kontaktstreifen 68 im Grund des U-Profils 74 verläuft. Ist das Stimulationskabel 24 von der Unipolar-Kanüle und deren Ansatz 12 getrennt, wie dies der Explosionsdarstellung der Figur 30 entspricht, so schützt das U-Profil 74 das freie blanke Ende des Kontaktstreifens 68 gegen eine unbeabsichtigte Berührung durch den Benutzer. Dadurch wird zuverlässig verhindert, dass der metallische Kontaktstreifen 68 den Benutzer verletzt oder dessen Handschuh beschädigt. Wird das Stimulationskabel 24 mit der Kunststoffhülse 70 in den Tunnel 64 des Befestigungsteils 26 eingesteckt, verläuft das Stimulationskabel 24 achsparallel zu dem Schlauch 14 des Kanülenrohrs 10. Das U-Profil 74 der Kunststoffhülse 70 ragt soweit in den Tunnel 64 des Befestigungsteils 26 hinein, dass der ausgewölbte Federarm 66 des Kontaktteils 38 von unten in das U-Profil 74 eingreifen kann und den Kontaktstreifen 68 unter dem elastischen Federdruck seiner Auswölbung zuverlässig kontaktiert. Damit ist die elektrisch leitende Verbindung von der Ader 25 des Stimulationskabels 24 über den Kontaktstreifen 68 und das Kontaktteil 38 zu dem Kanülenrohr 10 hergestellt.

Die sechste Ausführung hat den Vorteil, dass das Befestigungsteil 26 mit dem Kontaktteil 38 auch dann an dem Ansatz 12 befestigt bleiben kann, wenn das Stimulationskabel 24 nicht benötigt wird und nicht angeschlossen ist. Der Ansatz 12 mit dem Befestigungsteil 26 weist dadurch für den Benutzer dieselbe Form auf unabhängig davon, ob das Stimulationskabel 24 angeschlossen ist oder nicht. Wie bei allen Ausführungen überdeckt auch hier das an dem Ansatz 12 befestigte Befestigungsteil 26 das Kontaktteil 38 am Außenumfang vollständig elektrisch isolierend.

### Bezugszeichenliste

- 10: Kanülenrohr
- 12: Ansatz
- 14: Schlauch
- 16: Aussparung
- 18: Vorderer Flansch
- 20: Nut
- 22: Innenstufe
- 24: Stimulationskabel
- 25: Ader
- 26: Befestigungsteil
- 28: Rastarme
- 30: Nasen
- 32: Steg
- 34: Rille
- 36: Hinterer Flansch
- 37: Kerbe
- 38: Kontaktteil
- 40: Schneid-Klemm-Kontakt
- 42: Federzunge
- 44: Nuten
- 46: Lagerzapfen
- 48: Lagerpfanne
- 50: Rastarm
- 52: Vertiefung
- 54: Vorsprung
- 56: Einsenkung
- 58: Lagernase
- 60: Durchbruch
- 62: Federlappen
- 64: Tunnel
- 66: Federarm
- 68: Kontaktstreifen
- 70: Kunststoffhülse
- 72: Außenwulst
- 74: U-Profil

## Patentansprüche

1. Unipolar-Kanüle, mit einem metallischen Kanülenrohr (10), mit einem am proximalen Ende des Kanülenrohres (10) angebrachten Ansatz (12) aus einem elektrisch isolierenden Kunststoff, mit einem an dem Ansatz (12) angeordneten Anschluss zum Einleiten einer Flüssigkeit in das Kanülenrohr (10) und mit einem Stimulationskabel (24), welches mit einer elektrisch leitenden Ader (25) das Kanülenrohr (10) im Bereich des Ansatzes (12) elektrisch kontaktiert, wobei die Unipolar-Kanüle außerdem ein Befestigungsteil (26) aus einem elektrisch isolierenden Kunststoff umfasst, das seitlich an dem Ansatz (12) an dessen Außenumfang anliegend befestigbar ist, wobei das Befestigungsteil (26) ein die Ader (25) elektrisch kontaktierendes elektrisch leitendes Kontaktteil (38) in der Weise aufnimmt, dass das Kontaktteil (38) einerseits in eine Aussparung (16) des Ansatzes (12) eingreift und das Kanülenrohr (10) kontaktiert und andererseits nach außen durch das Befestigungsteil (26) vollständig isolierend abgedeckt wird, wenn das Befestigungsteil (26) an dem Ansatz (12) befestigt ist.

2. Unipolar-Kanüle nach Anspruch 1,
**dadurch gekennzeichnet, dass** das Befestigungsteil (26) mit einer elastisch federnden Schnappverbindung an dem Ansatz (12) befestigbar ist.

3. Unipolar-Kanüle nach Anspruch 2,
**dadurch gekennzeichnet, dass** das Befestigungsteil (26) mit wenigstens einem Paar von federnden Rastarmen (28) den Ansatz (12) umgreift.

4. Unipolar-Kanüle nach Anspruch 3,
**dadurch gekennzeichnet, dass** das Kontaktteil (38) zwischen den Rastarmen (28) angeordnet ist und die Rastarme (28) beiderseits der Aussparung (16) über den Ansatz (12) greifen.

5. Unipolar-Kanüle nach Anspruch 1 oder 2,
**dadurch gekennzeichnet, dass** das Befestigungsteil (26) mit seinem distalen Ende schwenkbar in den Ansatz (12) einsetzbar ist, dass das Befestigungsteil (26) gegen den Ansatz (12) schwenkbar ist und mittels der Schnappverbindung an dem Ansatz (12) anliegend befestigbar ist.

6. Unipolar-Kanüle nach einem der Ansprüche 1 bis 5,
**dadurch gekennzeichnet, dass** das Kontaktteil (38) einen Schneid-Klemm-Kontakt (40) aufweist, der auf das Kanülenrohr (10) gedrückt wird.

7. Unipolar-Kanüle nach einem der Ansprüche 1 bis 5,
**dadurch gekennzeichnet, dass** das Kontaktteil (38) eine Federzunge (42) aufweist, die sich außen an das Kanülenrohr (10) anlegt.

8. Unipolar-Kanüle nach einem der Ansprüche 1 bis 7,
**dadurch gekennzeichnet, dass** die Ader (25) des Stimulationskabels (24) mit dem in dem Befestigungsteil (26) aufgenommenen Kontaktteil (38) elektrisch leitend fest verbunden ist.

9. Unipolar-Kanüle nach einem der Ansprüche 1 bis 7,
**dadurch gekennzeichnet, dass** die Ader (25) des Stimulationskabels (24) mit dem in dem Befestigungsteil (26) aufgenommen Kontaktteil (38) elektrisch leitend steckbar verbindbar ist.

10. Unipolar-Kanüle nach einem der vorhergehenden Ansprüche,
**dadurch gekennzeichnet, dass** der Anschluss zum Einleiten der Flüssigkeit koaxial an den Ansatz (12) angesetzt ist.

11. Unipolar-Kanüle nach Anspruch 10 ,
**dadurch gekennzeichnet, dass** der Anschluss mit einem an den Ansatz (12) angesetzten Schlauch (14) ausgebildet ist.

## Claims

1. Unipolar cannula having a metallic cannula tube (10) with an attachment (12) made of an electrically isolating plastic arranged on the proximal end of the cannula tube (10), with a port arranged on the attachment (12) for introducing liquid into the cannula tube (10) and with a stimulation cable (24) that electrically contacts the cannula tube (10) in the region of the attachment (12) with an electrically conductive wire (25), wherein the unipolar cannula also has a mounting element (26) that is made of an electrically isolating material and that can be adjacently arranged on the attachment (12) laterally on its outer circumference, wherein the mounting element (26) receives an electrically conductive contact element (38) that contacts the wire (25) such that the contact element (38) engages in a recess (16) of the attachment (12) and contacts the cannula tube (10) on the one hand and, on the other hand, is entirely insulated from the outside by the mounting element (26) when the mounting element (26) is fastened to the attachment (12).

2. Unipolar cannula in accordance with claim 1,
**characterized in that** the mounting element (26) can be fastened to the attachment (12) with an elastically flexible snap connection.

3. Unipolar cannula in accordance with claim 2,
**characterized in that** the mounting element (26) grasps the attachment (12) with at least one pair of flexible locking arms (28).

4. Unipolar cannula in accordance with claim 3,
**characterized in that** the contact element (38) is arranged between the locking arms (28) and **in that** the locking arms (38) grasp on both sides of the recess (16) via the attachment (12).

5. Unipolar cannula in accordance with claim 1 or 2,
**characterized in that** the mounting element (26) can be swivelably inserted at its distal end into the attachment (12), **in that** the mounting element (26) can be swiveled towards the attachment (12) and can be tightly fastened to the attachment by means of the snap connection.

6. Unipolar cannula in accordance with any of claims 1 to 5,
**characterized in that** the contact element (38) comprises an insulation displacement contact (40) that is pressed onto the cannula tube (10).

7. Unipolar cannula in accordance with any of claims 1 to 5,
**characterized in that** the contact element (38) has a spring tongue that presses against the outside of the cannula tube (10).

8. Unipolar cannula in accordance with any of claims 1 to 7,
**characterized in that** the wire (25) of the stimulation cable (24) is fixedly and electrically conductively connected to the contact element (38) received by the mounting element (26).

9. Unipolar cannula in accordance with any of claims 1 to 7,
**characterized in that** the wire (25) of the stimulation cable (24) can be electrically conductively connected to the contact element (38) received by the mounting element (26).

10. Unipolar cannula in accordance with any of the previous claims, **characterized in that** the port for introducing the liquid is arranged coaxially on the attachment (12).

11. Unipolar cannula in accordance with claim 10,
**characterized in that** the port is implemented with a tube (14) arranged on the attachment (12).

## Revendications

1. Canule unipolaire comprenant un tube de canule métallique (10) ayant un embout (12) en un matériau synthétique électriquement isolant installé à l'extrémité proximale de ce tube de canule (10), une connexion permettant d'introduire un liquide dans le tube de canule (10) située sur l'embout (12) ainsi qu'un câble de stimulation (24) qui est en contact électrique avec un fil électriquement conducteur (25) du tube de canule (10) dans la zone de l'embout (12),
la canule unipolaire comportant en outre une pièce de fixation (26) en un matériau synthétique électriquement isolant qui peut être fixée latéralement sur l'embout (12) en étant appliquée sur sa périphérie externe, la pièce de fixation (26) recevant une pièce de contact (38) électriquement conductrice venant en contact électrique avec le fil (25) de sorte que la pièce de contact (38) d'une part, vienne, en prise dans un évidement (16) de l'embout (12) et vienne en contact avec le tube de canule (10), et, d'autre part, soit totalement recouverte en étant isolée vers l'extérieur par la pièce de fixation (26) lorsque cette pièce de fixation (26) est fixée à l'embout (12).

2. Canule unipolaire conforme à la revendication 1,
**caractérisée en ce que**
la pièce de fixation (26) peut être fixée sur l'embout (12) par une liaison par encliquetage à ressort.

3. Canule unipolaire conforme à la revendication 2,
**caractérisée en ce que**
la pièce de fixation (26) vient en prise autour de l'embout (12) par au moins une paire de bras d'encliquetage à ressort (28).

4. Canule unipolaire conforme à la revendication 3,
**caractérisée en ce que**
la pièce de contact (38) est située entre les bras d'encliquetage (28) et les bras d'encliquetage (28) viennent en prise de part sur l'embout (12) et d'autre de l'évidement (16).

5. Canule unipolaire conforme à la revendication 1 ou 2,
**caractérisée en ce que**
la pièce de fixation (26) peut être insérée dans l'embout (12) par son extrémité distale en étant susceptible de pivoter, la pièce de fixation (26) peut pivoter par rapport à l'embout (12) et peut être fixée en s'appliquant sur l'embout (12) au moyen de la liaison par encliquetage.

6. Canule unipolaire conforme à l'une des revendications 1 à 5,
**caractérisée en ce que**
la pièce de contact (38) comporte un contact de déplacement d'isolement (40) qui est comprimé sur le tube de canule (10).

7. Canule unipolaire conforme à l'une des revendications 1 à 5,
**caractérisée en ce que**
la pièce de contact (38) comporte une languette de contact (42) qui s'appuie extérieurement sur le tube de canule (10).

8. Canule unipolaire conforme à l'une des revendications 1 à 7,
**caractérisée en ce que**
le fil (25) du câble de stimulation (24) est relié solidairement de façon électriquement conductrice à la pièce de contact (38) logée dans la pièce de fixation (26).

9. Canule unipolaire conforme à l'une des revendications 1 à 7,
**caractérisée en ce que**
le fil (25) du câble de stimulation (24) peut être relié par enfichage de manière électriquement conductrice à la pièce de contact (38) logée dans la pièce de fixation (26).

10. Canule unipolaire conforme à l'une des revendications précédentes,
**caractérisée en ce que**
la connexion permettant l'introduction de liquide est installée coaxialement à l'embout (12).

11. Canule unipolaire conforme à la revendication 10,
**caractérisée en ce que**
la connexion est formée par un tuyau (14) monté sur l'embout (12).
